Europäisches Patentamt

European Patent Office

Office européen des brevets

(19)

(11) Publication number: **0 107 029**
**A1**

(12) **EUROPEAN PATENT APPLICATION**

(21) Application number: **83109249.9**

(22) Date of filing: **19.09.83**

(51) Int. Cl.³: **G 01 N 33/96**

(30) Priority: **30.09.82 US 428522**

(43) Date of publication of application: **02.05.84**
**Bulletin 84/18**

(84) Designated Contracting States: **DE FR GB**

(71) Applicant: **MILES LABORATORIES, INC., 1127 Myrtle Street, Elkhart Indiana 46514 (US)**

(72) Inventor: **Rapkin, Myron Coleman, 156 Briggs, Beaumont Texas 77707 (US)**
Inventor: **Tabb, David Lee, 2710 French Road, Beaumont Texas 77706 (US)**

(74) Representative: **Jesse, Ralf-Rüdiger, Dr. et al, Bayer AG Zentralbereich Patente Marken und Lizenzen, D-5090 Leverkusen 1 Bayerwerk (DE)**

(54) Control device and method for making same.

(57) A device is disclosed for preparing an analytical control solution of a substantially water-insoluble substance. The device comprises a base support member having affixed thereto a composition comprising a water-soluble polymer and the substance. The composition is adhered to the support member. Also disclosed is a method for preparing the device wherein a coating mixture containing the substance, an organic solvent, an etchant and a polymer soluble in both water and the solvent is applied to a base support member. A method for using the device to prepare the control solution comprises immersing the device into water for a time sufficient to enable the substance to become transferred from the device to the water.

## CONTROL DEVICE AND METHOD
## FOR MAKING SAME

## BACKGROUND OF THE INVENTION

*1. Field of the Invention*

This invention relates generally to analytical procedures for determining the presence and/or amount of an analyte in a solution, and more particularly to a device and method for preparing an analytical control solution containing a substantially water-insoluble substance which simulates an analyte. Such a solution is especially useful in the evaluation of analytical procedures for determining the presence and/or amount of sediment particles in aqueous suspension, such as in urine.

*2. Description of the Prior Art*

Analytical procedures for determining the presence of sample constituents, whether gravimetric, volumetric, spectrophotometric or the like, should be evaluated periodically for reliability of results. Unless the reliability of such procedures is assessed, any data developed from them is meaningless. Therefore, devising and maintaining an appropriate analytical procedure must necessarily include quality control measures, such as, an evaluation of experimental

MS-1252

error produced by the procedure, or an assessment of the training and efficiency of the technologist performing it. In other words, some means of predicting the dependability of the data produced by the analytical procedure, and the dependability of a person conducting it, is crucial to its successful use. In addition, it is desirable to have such controls available for training purposes.

The most efficacious and direct way to evaluate parameters such as reproducibility of results, sensitivity and accuracy of an analytical procedure, and to aid the training of technologists in the procedure, is to subject the procedure to a test sample wherein the presence and/or concentration of the substance being determined, or one which simulates the analyte, is known. Such a test sample is termed a control, and data furnished by the procedure can thereby be compared with known data to reveal discrepancies.

Exemplary of known analytical control devices is a product currently marketed by the Ames Division of Miles Laboratories, Inc., known as CHEK-STIX®. It consists of various pads of bibulous carrier mounted on a strip of plastic, each pad being impregnated with one or more substances which, when dissolved in water, simulate pathological urine constituents. Accordingly, when a CHEK-STIX device is immersed in a premeasured volume of water for a predetermined time, a control solution results which simulates urine containing such metabolites as glucose, bilirubin, urobilinogen, ketone, occult blood, nitrite and protein. Such a control solution can be used to monitor the test device known as N-MULTISTIX®, a dip-and-read analytical device also marketed by the Ames Division of Miles Laboratories, Inc.

MS-1252

Another example of a control device is embodied by the product TEK-CHEK®, also marketed by the Ames Division of Miles Laboratories, Inc. It comprises lyophilized urine containing natural and artificial additives to simulate both normal and pathological urine. In use, TEK-CHEK is added to a premeasured quantity of water to form the control solution.

A need has arisen for an efficient and inexpensive analytical control device which is suitable for use in the evaluation of procedures which are designed to detect the presence of substantially water-insoluble sediment components in body fluids such as urine. Such a device should be one which can be manufactured by relatively simple techniques using comparatively inexpensive components, and which, in use, will provide a reliable control solution to assure the accuracy of analytical procedures for determining sediment constituents in such fluids.

While the prior art control devices described *supra* do not include a component capable of producing a urine sediment control, conventional sediment control devices are known which include lyophilized natural urine in bulk or cake form. Lyophilized urine, however, is expensive to prepare, and the urine pools from which it is derived are subject to rapid biodegradation, thus increasing handling, storage and manufacturing costs. Moreover, lyophilized urines must be refrigerated in well-sealed containers prior to use, and are not readily adaptable to a format which will easily and conveniently provide control solutions containing sediment of desired types and amounts -- a quality necessary to simulate types of sedimentary material found in many pathological urines. It is, therefore, apparent that a primary goal to be achieved by an ideal sedi-

MS-1252

ment control device, especially one for urine sediment, is to provide a control solution which appears, to a skilled technologist examining it, to contain sediment particles which are virtually indistinguishable from those typically found in natural pathological urine samples. Such a control solution would be especially advantageous as a quality control tool, or as an aid in the evaluation and training of technologists. Moreover, because it is often of medical importance for a clinical diagnostician to be able to determine not only the presence or absence but also the amount of sedimentary particles in a urine sample, it is desirable that sediment control solutions, when used as critical diagnostic standards, contain known quantities of components of suitable appearance and size in order very closely to approximate natural sediment particles. Therefore, in order to provide an ideal control against which urine samples obtained for analysis can be compared, it is preferred that a control device be capable of delivering into a control solution predetermined amounts of particular sediment-simulating substances which appear to a trained observer to be actual sediment which would be expected to be present in a corresponding natural urine sample under study.

One approach to the preparation of a control solution designed to simulate the appearance of leukocytes in body fluids such as urine, blood, serum, spinal fluid or the like is disclosed in U.S. Patent No. 4,020,006 to Parker. Reconstituted body fluid is reportedly prepared by combining natural, lyophilized fluid, to which known amounts of particles have been added, with a suitable liquid. The particles, e.g., microspores or pollen, become dispersed throughout the reconstituted fluid and, so dispersed, are said to closely resemble leukocytes in body fluids.

MS-1252

The aforedescribed teachings of the prior art show that it has been contemplated to provide control solutions of many different analytes, or simulated analyte, as well as urine sediment, from devices which may comprise a carrier matrix or a lyophilized cake of natural urine components. Nevertheless, with respect to urine sediment controls, it is apparent that an ideal control device should provide a suitable control solution which accurately simulates sediment particle analytes in natural urine, while using a relatively simple, convenient construction capable of being easily manufactured. Such a device should provide a suitable control solution without the necessity for the use of natural urine components, filter paper carriers or other substrates containing an analyte material, or for expensive lyophilized urine. In addition, the device should enable substantially precise, measured amounts of materials simulating an analyte to be released into a control solution, a result not usually obtainable from devices using paper carriers.

## BRIEF DESCRIPTION OF THE INVENTION

The present invention is based upon the discovery of a new device and method for preparing a control solution containing a substantially water-insoluble analyte-simulating substance, as well as a novel method for making the device. In a preferred embodiment, the method comprises preparing a coating mixture by combining a substance which is capable of simulating a water-insoluble component found in natural urine sediment, an etchant substance, an organic solvent or carrier liquid and a polymeric material which is soluble both in water and in the organic solvent. The coating mixture is then applied to a portion of a

MS-1252

base support member; the support member comprises a substance capable of being etched by the etchant substance.  Finally, the coated base support member is dried.  A device according to the instant invention can comprise such a base support member having affixed thereto a composition capable , when immersed in water, of producing a suspension containing a substantially water-insoluble substance which simulates a component found in natural urinary sediment.  The composition comprises such a substance and a polymer which is soluble both in water and in an organic solvent, and is adhered to the base support member.

DETAILED DESCRIPTION OF THE INVENTION

A control device produced in accordance with the present invention is particularly useful for formulating control solutions containing simulated, substantially water-insoluble sediment in body fluids, for example, simulated sediment in urine.  Components of natural urine sediment which can be simulated by the invention include, for example, leukocytes, erythrocytes and casts.  However, it will be appreciated that while the instant invention is particularly described herein in terms of such a urine sediment control device, the principles of the invention are equally applicable to the preparation of control solutions containing substantially water-insoluble materials which are similar to naturally-occuring sedimentary components of various fluids, for example, body fluids from the stomach, spinal column and the like, and to the preparation of control solutions for materials which may occur in any particular fluid which may be the subject of an analytical procedure, therefore mandating a suitable control.  It may be seen, accordingly,

MS-1252

that the present control device, and a control solution produced therefrom, are not limited to the simulation of any particular analyte, but that many unrelated, substantially water-insoluble materials can be simulated by one of ordinary skill in the art, from devices produced according to the invention, when the disclosure hereof has been digested. It is, therefore, to be also appreciated that the determination of whether or not a particular material can be successfully simulated is fully within the capability of one of ordinary skill in the art, given the instant teachings and the broad range of substantially water-insoluble materials available which in physical characteristics may suitably approximate the presence and/or amount of a desired analyte in a solution.

In a preferred embodiment of the present invention, calcium oxalate (monohydrate) is used as a substance for simulating a component of urinary sediment when immersed in water to form a sediment suspension for a control solution. However, substances such as tanned red blood cells, real or simulated leukocytes and triple phosphate crystals are also suitable for this purpose. Also, it is contemplated by the invention that the above-named substances may be suitable for simulating other analytes in addition to urine sediment.

In the preparation of a control device according to the invention, the suitability of the substantially water-insoluble, analyte-simulating substance used in the device is governed by such considerations as particle size and shape and especially by appearance of the particles in a solution to a trained observer, for example as examined under a high power microscope. Because natural urine sediment is composed of such substances as triple phosphate crystals, the aforementioned

MS-1252

0107029

preferred device of the invention includes a sediment-simulating substance which possesses certain characteristics enabling it to closely approximate the appearance and amount of such actual sediment components, such as for example, refractile, rectangular crystals, similar in appearance to "coffin lids", and about 8 to 10 microns in length.

A device of the present invention can be prepared by forming a coating mixture and applying it to a base support member, such as an oblong plastic strip. The coating mixture comprises a solution or slurry, in a suitable organic solvent (i.e., carrier vehicle), of a water and solvent-soluble polymer, a substantially water-insoluble substance capable of simulating sediment in a solution and an etchant substance. In the coating mixture, the polymer serves also as a binder for the substance, essentially enabling formation of a suspension in which the sediment-simulating substance is dispersed, and which is capable of being coated to a desired extent and thickness upon the support member.

In use, to form a sediment control solution a device produced in accordance with the invention preferably is immersed in a predetermined quantity of water for a time sufficient for the coating upon the base support member to diffuse into the liquid, and with it the analyte-simulating substance to become transferred to the liquid. The control solution then is essentially a liquid suspension containing the substance as sediment, which thereafter can be centrifuged so that the sediment in the solution can be readily observed, such as under a microscope, for its similarity to a naturally occurring analyte under study.

A wide range of organic liquids are useful as suitable solvents, either alone or in mixtures. In many cases the etchant substance itself can be used

MS-1252

as the solvent. It will be apparent that the selection and quantity of an appropriate solvent, whether acetone, methanol, ethanol, xylene, benzene or other organic solvent or mixtures thereof, is a matter of choice which can be made through routine experimentation, considering the disclosure herein. The suitability and amount of a particular solvent to be used will ordinarily be governed only by the ability of the solvent to dissolve substantially completely the polymeric material used, and that it not dissolve or react with the sediment-simulating substance.

The water and organic solvent-soluble polymer used herein can include many compounds, provided it satisfies the aforementioned requirements of solubility, as well as being compatible with the other components of the coating mixture and of the ultimate device. Preferred polymers include ethyl hydroxylethylcellulose, guar gum, carboxymethyl cellulose, polyacrylamide, poly(diallyldimethylammonium chloride) and poly(ethyleneimine). Especially suitable and preferred is hydroxy propylcellulose, which is commercially available from Hercules, Inc., under the KLUCEL® trademark.

The etchant substance used for preparing the device is of a type and amount capable of at least partially etching the base support member without dissolving or distorting it during the application of the coating mixture thereto. For example, where the support member is composed of polystyrene, suitable etchant substances include toluene and benzene. In a preferred embodiment, the etchant substance is an aromatic liquid; especially preferred is toluene. The etchant can be incorporated in the coating mixture,

MS-1252

as described in the example, *infra*, of a preferred embodiment of the invention. Alternately, the etchant can be applied to the support member prior to application of the coating mixture thereupon, obviating the need for the inclusion of an etchant in the mixture prior to its being coated onto the support member.

The selection of material for use as the base support member is limited solely by its propensity to be etched by the etchant substance, and by its insolubility in water. Generally, however, it is preferred to use a rigid or semi-rigid homopolymer or copolymer material such as polystyrene, polyethylene and other polyolefins, nylon and others. In a preferred embodiment, the base support material is a biaxially-oriented polystyrene strip.

Because of the coaction between the etchant substance and the base support member, the coating mixture becomes adhered to the base support member. In a finished device of the invention, this highly desirable phenomenon enables excellent adhesion to the support member of the coating, and consequently of the sediment-simulating substance contained in the coating. This unexpectedly strong adhesion is believed to contribute to the enhanced ease of manufacture, and decreased cost of the present device, by comparison with known devices such as those previously described. Moreover, because the sediment-simulating substance is tightly bound in the coated film on the base support member, a device produced in accordance with the invention has excellent shelf life.

MS-1252

- 11 -

0107029

A urine sediment control solution which has been prepared from a device which was produced in accordance with the present invention, as described herein, has been found to contain sediment particles which, upon microscopic examination by a trained observer, appeared similar to particles usually found in some pathological urines.

Because the present invention enables the formation of urine sediment control solutions containing sediment particles in varying amounts -- the amount and extent of coating solution applied to the base support member being varied accordingly -- the similarity of the particles in a control solution to naturally occurring sediment is even more advantageously enhanced. It can be seen that this feature of the invention permits practically quantitative controls to be produced, containing predetermined amounts of a desired sediment-simulating substance, which can be especially advantageous for accurately approximating differing amounts of sediment which may be found in urine samples taken from patients having various pathological disorders. Moreover, such control of the amount and extent of the coating is possible during manufacture of a device of the invention, as exemplified in the Example, *infra*, through the use of conventional coating techniques.

Preferred ranges for the amount of the constituents of the coating mixture used in preparing a device of the invention include, by weight of the total mixture, from 1 to 20 percent polymer, from 75 to 98 percent solvent and from 1 to 5 percent etchant.

Although a preferred method for preparing a control device of the present invention is clearly set forth in the Example which follows, the basic procedure comprises forming the coating mixture, applying it to the base support member and drying the resulting composite. The coating mixture can be, and preferably is, applied to one end of an oblong-shaped support member, the other end serving as a handle. Preferably, a relatively large (as compared to the ultimate intended size of a single device) piece of support member, optimally a polystyrene sheet or roll, is coated in a strip along one of its long edges, by drawing the coating mixture out with a coating rod. Alternately, a doctor blade, coarse roller, brush or other suitable applicator can be used. After application, the coating solution is allowed to dry, and the composite is then split perpendicular to the coated stripe to provide devices wherein the sediment-simulating substance is at one end, the other functioning as a handle.

MS-1252

EXAMPLE

The following example is provided in order to set forth the presently envisioned best mode of making and using the instant invention. The example is intended only to illustrate and disclose, and is in no way to be interpreted as limiting the scope of the invention.

A control device for producing a control solution for simulating sediment in urine was prepared for use in accordance with the present invention by initially preparing a coating solution. The coating solution was prepared by combining in a 100 milliliter (ml) volumetric flask a 1.0 gram (g) quantity of hydroxy propylcellulose (KLUCEL, obtained from Hercules, Inc. ) with approximately 60 ml of a solvent blend comprising the following ingredients:

|           |         |
|-----------|---------|
| Acetone   | 49.5 ml |
| Methanol  | 49.5 ml |
| Toluene   | 1.0 ml  |

The ingredients in the flask were stirred together until it was observed that all the hydroxy propylcellulose had dissolved. Thereafter the remainder of the solvent blend (40 ml) was added to the flask.

A 1.0 ml portion of the coating solution, prepared as described above, was placed in a test tube, and 0.05 g of calcium oxalate (monohydrate) was added to the tube. The mixture in the tube was stirred for approximately 30 seconds using a wood applicator stick and then was poured onto a sheet of polystyrene (TRYCITE®, commercially available from American Can Co.,

MS-1252

Neenah, Wisconsin) measuring approximately 4 inches by 10 inches, which served as a base support member. The coating mixture was then spread upon the polystyrene sheet, to a thickness of approximately 0.8 mil, with an applicator known as a Mayer rod.*

The coated polystyrene was allowed to air-dry for approximately ten minutes at room temperature, whereafter a piece of the coated sheet approximately 1.0 centimeter (cm) in width x 4.0 inches in length, and containing a coated area approximately 1.0 cm wide and 2.5 cm long, was cut and placed in a test tube containing about 15 ml of distilled water. The tube was capped and shaken for about 1 minute and then centrifuged for about 5 minutes at 2000 revolutions per minute (rpm). After centrifuging, the supernatant was decanted and the sediment which appeared in the bottom of the tube was resuspended by gently tapping the edge of the tube. A drop of the sediment was then removed, placed on a microscope slide and examined under high power for its similarity to natural urine sediment. The sediment, in a crystalline form, appeared under the microscope to bear a close resemblance to, and was most virtually indistinguishable from, natural urine sediment crystals which are often found in clinically pathological urine samples.

*The particular rod used was designated "No. 12", and is obtainable from R.D.Specialties, Inc., Webster, N.Y. Such rods are frequently used in the coating industry, and comprise a steel rod wrapped tightly with varying gauges of stainless steel wire. The thickness of an applied coating is dependent upon the diameter of the wrapping wire; the distance between complete winds of the wiring enables a predetermined amount of coating to be deposited. The approximated thickness of the coating deposited can be calculated by multiplying the rod number by a factor of 0.09 to give the coating thickness in mils. The rod numbers reportedly available range from 3 to 75, providing coating thicknesses of from about 0.27 mil to 6.75 mils.

MS-1252

It will be appreciated that, while a particular amount and extent of a coating mixture has been described in the foregoing example as being applied upon a base support member to produce a control device according to the invention, in the manufacture of such devices the coating solution can be spread over any desired surface area to any particular thickness, depending upon the conventional method and means for coating employed. It will be apparent to one skilled in the art that the choice of such parameters as the surface area and thickness of the coating on the support member will be governed by the selection of the predetermined amount of sediment-simulating substance which is desired in the ultimate control solution produced by immersing a device of the invention in water.

Although preferred embodiments of the present invention have been described, other embodiments and modifications thereof will readily become apparent from the disclosure hereof. The invention, therefore, is not limited thereto or thereby, and has a scope defined only by the following claims.

MS-1252

CLAIMS:

1. A device for preparing an analytical control solution containing a substantially water-insoluble substance, said device comprising a base support member having affixed thereto a composition comprising a water-soluble polymer and the substance, said composition being adhered to the surface of said support member.

2. A device according to claim 1, characterized in that the polymer is hydroxy propylcellulose, ethyl hydroxyethylcellulose, guar gum, carboxymethyl cellulose, polyacrylamide, poly(diallyl-dimethylammonium chloride) or poly(ethylene-imine).

3. A device according to claim 1 or 2, characterized in that the substance comprises a material which is capable of simulating sediment in urine and preferably is calcium oxalate.

4. A device according to any one of claims 1 through 3, characterized in that the support member is polystyrene.

5. A method for making a device for preparing an analytical control solution containing a substantially water-insoluble substance, which method comprises the steps of:
   a) preparing a coating mixture by combining the substance, an etchant, an organic solvent, and a polymeric material which is soluble in both water and the solvent;

MS-1252

- 17 -

0107029

b) applying the coating mixture to a base support member capable of being at least partially etched by the etchant to form a composite; and

c) drying the composite.

6. A method according to claim 5, characterized in that the polymer is hydroxy propylcellulose, ethyl hydroxyethylcellulose, guar gum, carboxymethyl cellulose, polyacrylamide, poly(diallyldimethylammonium chloride) or poly (ethylene-imine).

7. A method according to claim 5 or 6, characterized in that the organic solvent comprises a mixture of acetone and methanol.

8. A method according to any of claims 5 to 7, characterized in that the etchant is an aromatic solvent, preferably toluene.

9. A method according to any of claims 5 to 8, characterized in that the support member is polystyrene.

10. A method according to any one of claims 5 through 9, characterized in that the substance comprises a material which is capable of simulating sediment in urine, and preferably is calcium oxalate.

MS-1252

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A,D | US-A-4 020 006 (J.E. PARKER) <br> * Claims 1, 6, 7 * | 1,3 | G 01 N 33/96 |
| A,P | EP-A-0 068 379 (MILES LABORATORIES INC.) <br> * Claims 1, 2 * | 2 | |
| A | DE-A-2 835 296 (MILES LABORATORIES INC.) <br> * Claims 1, 2 * | 1 | |
| A | DE-A-2 721 681 (BEHRINGWERKE AG) | | |

--- ---

---

TECHNICAL FIELDS SEARCHED (Int. Cl. ³)

G 01 N 33/96

The present search report has been drawn up for all claims

| Place of search <br> BERLIN | Date of completion of the search <br> 20-12-1983 | Examiner <br> SCHWARTZ K |
|---|---|---|